# EUROPEAN PATENT SPECIFICATION

(11) **EP 4 380 870 B1**
(45) Date of publication and mention of the grant of the patent: **23.09.2026**
(21) Application number: 22851491.5
(22) Date of filing: 05.08.2022
(51) Int. Cl.: B65D 5/74, B65D 5/76, A61M 15/00, A61M 11/02, A61M 16/06

(54) **A MEDICATION INHALATION DEVICE**
VORRICHTUNG ZUR INHALATION VON MEDIKAMENTEN
DISPOSITIF D'INHALATION DE MÉDICAMENT

(30) Priority: 05.08.2021 AU 2021902425
(43) Date of publication of application: 12.06.2024
(73) Proprietor: Bird Healthcare Pty Ltd, Bayswater, VIC 3153 (AU)
(72) Inventor: BIRD, Nicholas, Bayswater, Victoria 3153 (AU); BOULT, Timothy, Bayswater, Victoria 3153 (AU); GOVER, Neale, Bayswater, Victoria 3153 (AU); HABERFIELD, David, Bayswater, Victoria 3153 (AU)
(74) Representative: Nederlandsch Octrooibureau
(86) International application number: PCT/AU2022/050853
(87) International publication number: WO 2023/010180

(56) References cited:
- US-A1- 2002 129 814
- US-A1- 2009 032 019
- US-A1- 2010 163 045
- US-A1- 2010 269 819
- US-A1- 2019 151 578
- US-A1- 2019 358 415
- US-B1- 6 202 643
- US-B2- 8 413 651

## Description

### FIELD OF THE INVENTION

The invention relates to medication inhalation devices, such as spacers configured for use with meter-dose inhalers (MDI).

The invention also relates to blanks for making medication inhalation devices.

The invention also relates to methods of making medication inhalation devices using blanks.

### BACKGROND ART

Medication delivery devices are commonly used for the treatment of breathing conditions, such as asthma.

There are many different types of medication delivery devices, including: meter-dosed inhalers (MDI) ; dry powder inhalers (DPI); soft mist inhalers (SMI); and nebulizers.

Medication delivery devices work by delivering a medication, typically comprising a steroid or a bronchodilator, into the lungs of a patient. The medication relieves the symptoms of asthma, which include breathlessness and having wheezy or tight-chested breathing. Medication delivery devices can also be used to deliver medication for other respiratory conditions.

Nebulisers work by changing a liquid medication into a vapour phase that can be inhaled by a patient. Nebulisers work by pumping pressurised air through a liquid medication to form a fine mist, which is then breathed in through a mask or mouthpiece.

The most common type of medication delivery device is the MDI, known colloquially as a "puffer".

A MDI is made up of three standard components: a pressurised canister; an actuator; and a metering valve.

The medication is typically stored in solution in the pressurised canister. The pressurised cannister contains the medication, a liquefied gas propellant and, in many cases, stabilising excipients. The pressurised canister is attached to the actuator. On activation of the actuator by a person, the MDI releases a fixed dose of medication in aerosol form through the metering valve and into the person's lungs.

Medication delivery devices require significant coordination as a person must discharge the medication at or near the same time that they inhale in order for the medication to be effective.

To increase the effectiveness of MDIs, a medication inhalation device, such as a "spacer", is often used.

Spacers help delivery of the medication to the lungs and small airways, with less medication ending up in the mouth and throat - which can lead to irritation or mild infections. A spacer can also make it easier to coordinate breathing in and actuating the inhaler.

Spacers typically comprise a tube or a chamber which adds space between the mouth and canister of medication. The spacer has an inlet opening configured to receive a mouthpiece of an inhaler and an outlet opening that is received in the mouth of the person.

The invention provides alternative spacers to spacers known to the applicant.

The above references to the background art do not constitute an admission that the art forms a part of the common general knowledge of a person of ordinary skill in the art. The above references are also not intended to limit the application of the device and method as disclosed herein.

In US 2010/0163045 A1, a device, system, and method are provided for dispersing an aerosol formulation comprising a medicament. The device may be useful in administering inhalable vaccines and the like. The device is designed for low cost and is applicable to a multitude of patient populations. The device has a spacer (10) that is suitable for use with a wide range of aerosolised formulations, particularly powders. The spacer (10) may be an expandable volume spacer (10). The spacer (10) may also be used with other inhalation aids (80). The spacer (10) may include a multi-purpose outlet (40) for use with different groups within the target patient population.

US 6,202,643 Bl describes a medication inhalation apparatus for use with an MDI inhaler that includes an elongated housing for receiving a plume of medication particles ejected by the MDI inhaler, a mouthpiece, and an inhalation valve disposed between the mouthpiece and the housing. An exhalation port or valve in the mouthpiece allows exhalation through the mouthpiece, presenting little resistance to the exhalation effort of the patient. An adapter receives and stabilises a mouthpiece of the MDI inhaler. The inhalation valve includes an inhalation flap hanging adjacent to a valve seat. Exhalation into the mouth- piece presses the inhalation flap against the valve seat, forcing exhaled gas through the exhalation port or valve. Inhalation causes the inhalation flap to swing away from the valve seat to open a path for the medication plume. In one embodiment, the entire inhalation apparatus is constructed from a single sheet of foldable sheet material.

### SUMMARY OF THE INVENTION

The invention is defined by the appended claims. Subject-matter which does not fall under the scope of the claims is not part of the invention.

The applicant has developed two types of medication inhalation devices that are suitable, by way of example, for use with "spacers". One device is multi-functional in that it is configured for use in a mouthpiece configuration and a mask configuration. The other device is configured for use in a mask configuration only.

The invention provides a medication inhalation device comprising a housing that is movable between a collapsed state and an expanded state, wherein when the housing is in the expanded state the housing defines a volume in which medication (such as in the form of medication particles), delivered (for example, ejected) from a medication delivery device can mix with air, and wherein when the housing is in the expanded state the housing comprises:
external walls that define the volume,
a first end with an inlet opening adapted to receive a medication from a medication delivery device for delivering the medication to the volume, and
a second end with an outlet opening adapted to communicate with a mouth of a user such that a mixture of air and medication can be inhaled from the volume by the user,
the second end being convertible between a mouthpiece configuration and a mask configuration,
wherein, in the mouthpiece configuration, the mouth of the user can be placed in relation to the outlet opening so that the mixture of air and medication can be inhaled, and
wherein, in the mask configuration, the outlet opening envelops the mouth and a nose of the user such that the second end forms a sealing relationship with the face of the user so that the mixture of air and medication can be inhaled.

The invention also provides a medication inhalation device comprising a housing that is movable between a collapsed state and an expanded state, wherein when the housing is in the expanded state the housing defines a volume in which medication (such as in the form of medication particles), delivered (for example, ejected) from a medication delivery device can mix with air, and wherein when the housing is in the expanded state the housing comprises:
external walls that define the volume,
a first end with an inlet opening adapted to receive a medication from a medication delivery device for delivering the medication to the volume, and
a second end with an outlet opening that is configured to envelop a mouth and a nose of a user such that the second end forms a sealing relationship with the face of the user so that the mixture of air and medication can be inhaled from the volume by the user.

The medication delivery device may be any medication delivery device known in the art, for example: a meter-dosed inhaler (MDI); a dry powder inhaler (DPI); a soft mist inhaler (SMI); or a nebulizer. It is also envisaged the medication delivery device may be a bespoke piece of equipment. To this end, the inlet opening may be configured to universally receive an outlet of any one of the above described medication delivery devices.

An advantage of the mouthpiece/mask device of the invention is that it is adapted for use in two configurations, i.e. in the mouthpiece configuration and the mask configuration. This provides options for users.

An advantage of the mouthpiece/mask device of the invention is that a one-size unit is suitable for use by people of different sizes and/or ages, e.g. adults and children, due to being convertible between a mouthpiece configuration and a mask configuration.

An advantage of the mouthpiece/mask device and mask only device of the invention is that the mask allows the device to be used with children under 5 years old.

The medication may be any suitable medication in any suitable form.

The medication may be in the form of medication particles.

The medication may comprise a steroid or a bronchodilator.

In the collapsed state, the above-described housings may have a substantially flat configuration.

The housings may comprise an internal wall which extends between the external walls to partition the volume into two chambers when in the expanded state, with the internal wall having an opening to allow fluid flow between the chambers.

The housings may have at least one one-way valve.

The housings may have a one-way inhalation valve and a one-way exhalation valve.

The one-way inhalation valve may be located on the internal wall.

The one-way exhalation valve may be located on the external wall.

The one-way valve may comprise a moveable flap. In one example, the one-way valve comprises a body having a hole and a flap that is pivotably connected to a body along an edge of the flap, the flap being pivotable between a closed position in which the flap covers the hole to substantially restrict air flow therethrough and an open position in which the flap uncovers the hole to allow substantially unrestricted air flow therethrough.

The one-way valve may be a flutter valve. In one example, the flutter valve comprises a body having a hole and a resilient sleeve connected to the body, the sleeve is movable between a deflated position in which air flow through the sleeve is substantially restricted and an inflated position in which air flow through the sleeve is substantially unrestricted.

The one-way valve increases the efficiency of delivery of inhaled medicine as the direction of flow of the medicine is controlled.

The housings may have a viewing window.

The viewing window may be made from clear plastic, such as polyethylene or PVC.

The viewing window allows a user to visually check inside the housings to make sure there are no obstructions that would otherwise hinder inhalation of the mixture of air and medication particles.

The device may be formed from a single unitary sheet of stock.

The sheet may be made from paper or cardboard.

Forming the device from a single unitary sheet of stock simplifies the assembly process, as it minimises the number of separate components that need to be assembled together.

Paper or cardboard are relatively inexpensive and recyclable materials and therefore the device may be suitable for a single use application. Single use applications have the advantage that they do not require cleaning.

The housings may be tapered between the first end and the second end.

The tapered housings promote turbulent conditions within the volume which assist in mixing the medication particles with air.

The invention also provides a blank for making a medication inhalation device, the blank being made from a foldable board and comprising:
a plurality of panels separated by fold lines that allow the panels to be folded about the fold lines to form the device, with the formed device defining a housing that is movable between a collapsed state and an expanded state, and the expanded state being convertible between a mouthpiece configuration and a mask configuration, the panels comprising:
a central panel and a plurality of adjacent panels to the central panel,
the adjacent panels comprising:
   a first adjacent panel having a first opening,
   a second adjacent panel having a second opening and a separable portion;
   wherein the adjacent panels are foldable relative to the central panel about the fold lines and can be secured to the central panel to define external walls of a housing in the formed device,
   wherein the housing defines a volume when the housing is in the expanded state of the formed device and medication, such as in the form of medication particles, can be delivered (for example, ejected) to the volume from a medication device communicating with the first opening and can mix with air and be inhaled by a user through the second opening, with the second opening forming the mouthpiece configuration of the formed device, and
   wherein the separable portion can be separated from the formed device and increase the size of the second opening and form the mask configuration when the housing is in the expanded state of the formed device, with the mask configuration being able to envelop a mouth and a nose of the user and form a sealing relationship with the face of the user so that air and medication can be inhaled from the volume.

The invention also provides a blank for making a medication inhalation device, the blank being made from a foldable board and comprising:
a plurality of panels separated by fold lines that allow the panels to be folded about the fold lines to form the device, with the formed device defining a housing that is movable between a collapsed state and an expanded state, the panels comprising:
a central panel and a plurality of adjacent panels to the central panel,
at least one of the adjacent panels having a first opening,
wherein the adjacent panels are foldable relative to the central panel about the fold lines and can be secured to the central panel to define external walls of the housing in the formed device,
wherein the formed device has a second opening configured to envelop a mouth and a nose of a user and the housing defines a volume, when the housing is in the expanded state of the formed device, and medication, such as in the form of medication particles, can be delivered (for example, ejected) from a medication delivery device communicating with the first opening and can mix with air in the volume and be inhaled by the user via the second opening enveloping the mouth and the nose of the user and forming a sealing relationship with the face of the user.

The first adjacent panel of the above-described blanks may comprise a tongue portion having a third opening, the tongue portion being inwardly foldable about fold lines to define an internal wall which extends between, and securable to, the external walls to partition the volume into two chambers in which the third opening allows fluid flow between the chambers.

The third opening may have a one-way valve.

The one-way valve may comprise a moveable flap.

The one-way valve may comprise a body having a hole and a flap that is pivotably connected to a body along an edge of the flap, the flap being pivotable between a closed position in which the flap covers the hole to substantially restrict air flow therethrough and an open position in which the flap uncovers the hole to allow substantially unrestricted air flow therethrough.

The one-way valve may be a flutter valve.

The flutter valve may comprise a body having a hole and a resilient sleeve connected to the body, the sleeve is movable between a deflated position in which air flow through the sleeve is substantially restricted and an inflated position in which air flow through the sleeve is substantially unrestricted.

The one-way valve increases the efficiency of delivery of inhaled medicine as the direction of flow of the medicine is controlled.

The central panel may have a viewing window.

The viewing window may be made from clear plastic, such as polyethylene or PVC.

The viewing window allows a user to visually check inside the housing to make sure there are no obstructions that would otherwise hinder inhalation of the mixture of air and medication particles.

The separable portion may comprise perforations, such as in the form of tear lines.

The invention also provides a method of making the medication inhalation device defined above using the blank defined above, the method comprising the steps of:
- folding the plurality of adjacent panels relative to the central panel about the fold lines;
- securing the adjacent panels to the central panel to define external walls of the housing; and
- breaking the separable portion to convert the second end from the mouthpiece configuration to the mask configuration.

The invention also provides a method of making the medication inhalation device described above using the blank described above, the method comprising the steps of:
- folding the plurality of adjacent panels relative to the central panel about the fold lines; and
- securing the adjacent panels to the central panel to define external walls of the housing.

The step of securing may be achieved by any conventional means known in the art.

In one embodiment, each adjacent panel has double-sided adhesive tape.

In another embodiment, each adjacent panel comprises a tongue that is inserted into and frictionally retained in a slot on the central panel.

In another embodiment, each adjacent panel comprises a portion covered in Velcro^{®} that can be attached to a corresponding portion on the central panel that is covered in Velcro^{®}.

The step of removing the removable portion may be achieved by any conventional means known in the art. In one preferred embodiment, at least one of the panels has a weakened region. The weakened region may be formed by perforations in the panel.

### BRIEF DESCRIPTION OF THE DRAWINGS

Notwithstanding any other forms which may fall within the scope of the device as set forth in the Summary of the Invention, specific embodiments of the invention will now be described, by way of example only, with reference to the accompanying drawings in which:
Figure 1 is a front perspective view of a medication inhalation device according to an embodiment of the invention with the housing in a mouthpiece configuration.
Figure 2 is an enlarged rear view of the medication inhalation device of Figure 1 showing the inlet opening.
Figure 3 is a front perspective view of the device shown in Figure 1 with the housing in a mask configuration.
Figure 4 is a front view of the device shown in Figure 3, i.e. in a mask configuration.
Figure 5 is a plan view of the device shown in Figure 1.
Figure 6 is a side sectional view of the device shown in Figure 1, i.e. in a mouthpiece configuration.
Figure 7 is a side sectional view of the device shown in Figure 3, i.e. in a mask configuration.
Figure 8 is a plan view of a blank used to make the device shown in Figure 1.
Figure 9 is a plan view of a blank according to another embodiment of the invention which in the assembled device forms a mask.

### DETAILED DESCRIPTION OF EMBODIMENTS

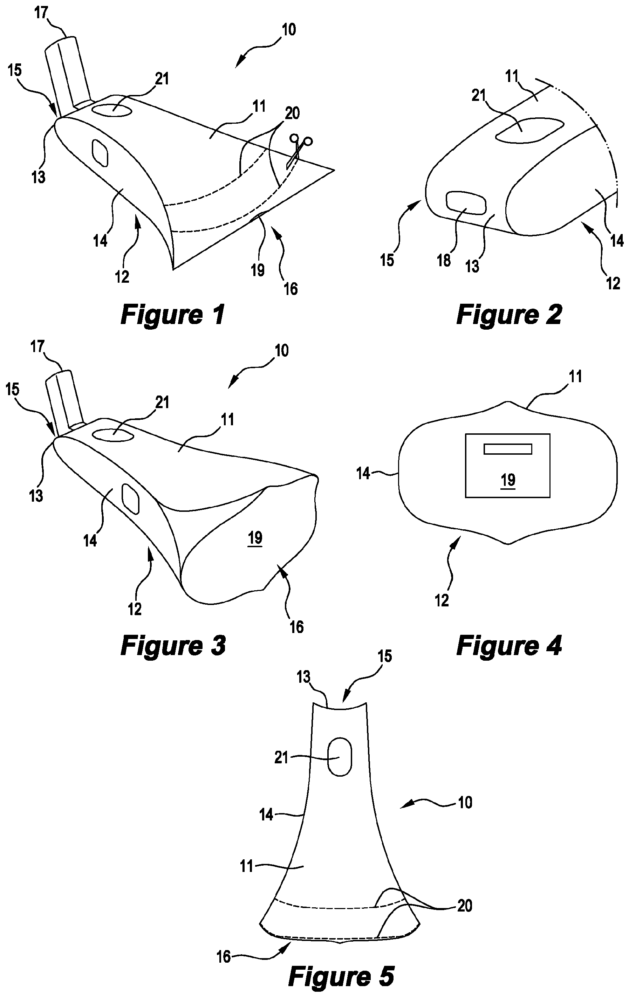

Figures 1-7 show a medication inhalation device according to an embodiment of the present invention.

As shown in Figure 1, the device comprises a housing 10 having external walls (upper wall 11, lower wall 12, end wall 13 and side walls 14) that define a volume. The housing 10 is elongate and tapered between the end wall 13, that forms a first end 15 of the device, and a second end 16. At this end, the upper and lower walls 11, 12 converge together - there is a junction of the upper wall 11 and the lower wall 12.

As shown in Figure 2, a first opening 18 is located on the end wall 13. The first opening 18 is shaped and dimensioned to insertably receive a mouthpiece of a medication delivery device, such as a meter dose inhaler 17. The first opening 18 is herein referred to, in this embodiment, as a medicated particle inlet opening 18 as its function is to allow a plume of medicated particles to enter the housing 10 from the meter dose inhaler 17.

Turning back to Figure 1, a second opening 19 is located at the junction of the upper wall 11 and the lower wall 12. The second opening 19 has an area that is sized to allow a mouth of a user to communicate therewith in order for the user to inhale a mixture of air and medication particles from the volume. The second opening 19 is also referred to as a mouthpiece outlet opening 19 as its function is to allow the mixture of air and medication particles to exit the housing 10 into the user's mouth.

The housing 10 is convertible between a mouthpiece configuration with the outlet opening 19 as shown in Figure 1 and a mask configuration as shown in Figures 3 and 4.

Figure 1 shows that "cut lines" 20 are printed or otherwise formed on at least the upper wall 11. The Figures show two cut lines 20, spaced apart along the length of the upper wall 11. It can be appreciated from Figures 1 and 3 that cutting or tearing along one of the cut lines 20 removes an end section of the housing 10 at the second end 16 of the device shown in Figure 1, with the result that the second end 16 can be opened outwardly to form the mask configuration shown in Figures 3 and 4. Specifically, once separated, the side walls 14 can be squeezed inwards to cause the upper and lower walls 11, 12 to move outwardly away from each other to shape the second end 16 such that it produces the mask configuration opening 19 (as shown in Figures 3 and 4) that has a significantly greater area than that of the outlet opening 19 (as shown in Figure 1) and can be placed over to envelop the mouth and nose of the user. In such a configuration, the second end 16 forms a sealing relationship with the face of the user so that the mixture of air and medication particles can be inhaled.

An advantage of converting the housing between the mouthpiece configuration and the mask configuration is that it provides options for users. The device also provides a one-size unit that is suitable for use by people of different sizes and/or ages, e.g. adults and children. In particular, the mask configuration allows the device to be used with children under 5 years old without the need for a separate mask.

Figure 5 shows the elongate and tapered shape of the housing 10 the first end 15 and the second end 16. The tapered shape promotes turbulent conditions within the volume which assist in mixing the medication particles with air.

As can be seen from Figure 5, the upper wall 11 comprises a viewing window 21 made from clear plastic, such as polyethylene or PVC. The viewing window 21 allows a user to visually check inside the housing to make sure there are no obstructions that would otherwise hinder inhalation of the mixture of air and medication particles.

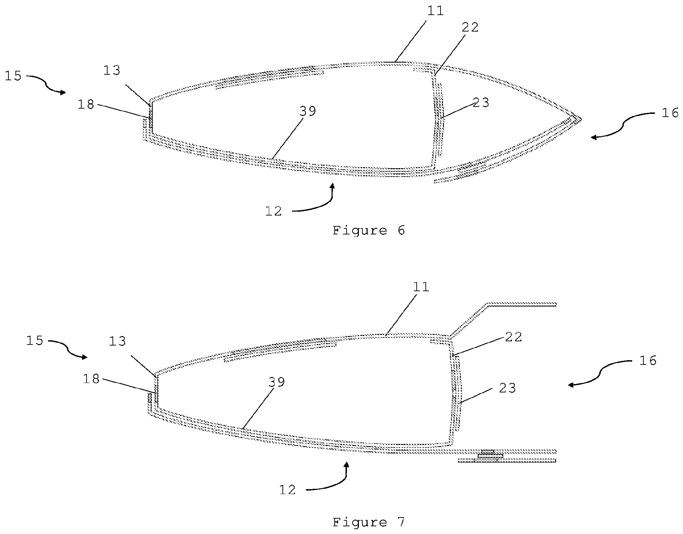

The device also includes an internal wall 22. As can best be seen in Figures 6 and 7, the internal wall 22 extends between the external walls 11, 12, 13, 14 to partition the volume into two chambers, with the internal wall 22 having an opening to allow fluid flow between the chambers. The opening has a one-way valve 23 to allow substantially unrestricted air flow in a first direction and substantially restrict air flow in a second direction, opposite to the first direction.

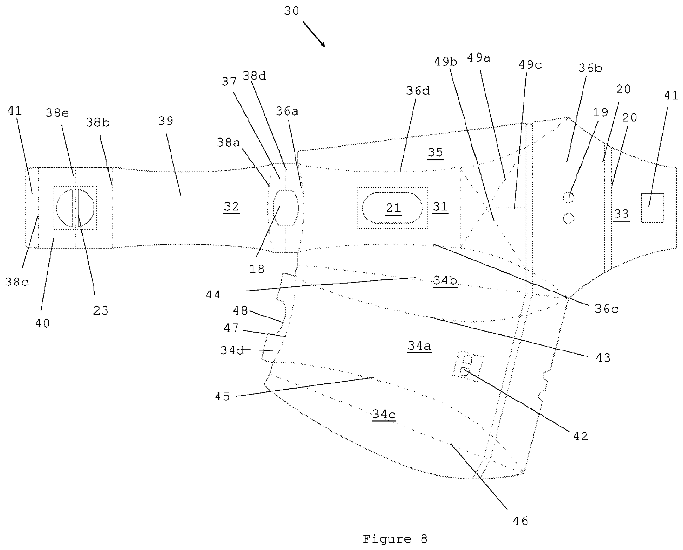

Figure 8 shows a blank 30 for making a medication inhalation device according to an embodiment of the present invention.

The blank 30 is formed from a foldable board and includes a plurality of panels separated by fold lines.

More particularly, the blank 30 includes a central panel 31, a first adjacent panel 32, a second adjacent panel 33, a third adjacent panel 34 and a fourth adjacent panel 35 all of which are separated by respective fold lines 36a, 36b, 36c, 36d.

The first adjacent panel 32 comprises the first medicated particles inlet opening 18.

The second adjacent 33 panel comprises the second outlet, i.e. mouthpiece, opening 19.

The adjacent panels 32, 33, 34, 35 are foldable relative to the central panel 31 about a respective fold line in the first set of fold lines 36a, 36b, 36c, 36d and can be secured together (adhered or otherwise) to form, together with the central panel 31, the external walls of the housing 10.

The central panel 31 comprises the viewing window 21 and a set of fold lines for assisting in contouring the second end of the housing to the shape of a user's nose. The set of fold lines comprises a pair of fold lines 49a, 49b that define a "X" shape and a fold line 49c that intersects with a central point of the "X" shape.

The first adjacent panel 32 has four sub-panels separated by fold lines. There is:
- A first sub-panel 37 that is separated from the central panel 31 by the curved fold line 36a and, in the assembled device forms the end wall 13 with the medicated particles inlet opening 18. The first sub-panel 37 can also be folded in half about the fold line 38d.
- An adjacent tongue-shaped sub-panel 39 separated from the first sub-panel 37 by a curved fold line 38a.
- An internal wall sub-panel 40 separated from the tongue-shaped sub-panel 39 by a straight fold line 38b and, in the assembled device forms the internal wall 22 (see Figures 6 and 7). The internal wall sub-panel 40 can also be folded in half about the fold line 38e.
- A fixing tab sub-panel 41 separated from the internal wall sub-panel 40 by a straight fold line 38c. The internal wall sub-panel 40 comprises the one-way valve 23.

The second adjacent panel 33 is separated from the central panel 31 by a straight fold line 36b. On an underside surface of the second adjacent panel 33 is a fixing portion 41 that is configured to engage with and be secured to a corresponding fixing portion 42 on the third adjacent panel 34. This can be achieved by any means known in the art for example using adhesives, such as double sided sticky tape, or Velcro^{®} hook/loop fasteners.

The third adjacent panel 34 has four sub-panels separated by fold lines. There is:
- A main sub-panel 34a comprising the corresponding fixing portion 42 (as previously described).
- A gusset sub-panel 34b that is located between the main sub-panel 34a and the central panel 31. The gusset sub-panel is separated from the main sub-panel 34a by a curved fold line 43 and is also separated from the central panel 31 by a curved fold line 36c. The gusset sub-panel 42 in the assembled device forms one of the side walls 14. The gusset sub-panel 34b also has a straight fold line 44 located between the fold lines 36c and 43.

- A side sub-panel fixing tab 34c located along a side edge of the main sub-panel 34a. The side sub-panel fixing tab 34c is separated from the main sub-panel 34a by a curved fold line 45. The side sub-panel fixing tab 34c also has a straight fold line 46 that is located between a free edge of the side sub-panel fixing tab 34c and the curved fold line 45.
- A rear sub-panel fixing tab 34d located along a rear edge of the main sub-panel 34a and separated from the main sub-panel 34a by a curved fold line 47. The rear sub-panel fixing tab 34d comprises a cut-out 48 which conforms to the shape of the inlet opening 18.

The fourth adjacent panel 35 is separated from the central panel 31 by a curved fold line 36d. The fourth adjacent panel 35 together with the side sub-panel fixing tab 34c form one of the side walls of the assembled device 10.

The method of assembling the blank 30 into the device 10 comprises the following steps:
1. The first adjacent panel 32 is folded relative to the central panel 31 about the curved fold line 36a.
2. The tongue-shaped sub-panel 39 is folded inwards about the curved fold line 38a to form part of the lower wall 12 of the housing 10 - see Figures 6 and 7. The first sub-panel 37 forms part of the rear wall 13 of the housing 10.
3. Once folded as described, the internal wall sub-panel 40 is folded 90° back on itself via the fold line 38b so that the end sub-panel 40 extends between the lower wall 12 and the upper wall 11 in the assembled device.
4. The fixing tab sub-panel 41 is folded relative to the internal wall sub-panel 40 about the fold line 38c.
5. The fixing tab sub-panel 41 is secured (adhered or otherwise) to an underside surface of the central panel 31.
6. The side sub-panel fixing tab 34c is folded relative to the main sub-panel 34a about the fold line 45.
7. The gusset sub-panel 34b is folded relative to the central panel 31 about the curved fold line 36c.
8. The main sub-panel 34a is folded relative to the gusset sub-panel 34b about the curved fold line 43.
9. The fourth adjacent panel 35 is folded relative to the central panel 31 about the curved fold line 36d.
10. The side sub-panel fixing tab 34c is secured (adhered or otherwise) to the underside surface of the fourth adjacent panel 35.
11. The second adjacent panel 33 is folded relative to the central panel 31 about the fold line 36b such that it overlaps the now secured third adjacent panel 34.
12. The fixing portion 41 on the underside surface of the second adjacent panel 33 is secured to the corresponding fixing portion 42 on the third adjacent panel 34.
13. The rear sub-panel fixing tab 34d is folded relative to the main sub-panel 34a about the fold line 47.
14. The rear sub-panel fixing tab 34d is secured (adhered or otherwise) to the first sub-panel 37 to complete the rear wall 13.

Now the blank 30 has been assembled into the medication inhalation device 10, the housing can be collapsed in a flat pack configuration by folding the respective panels about fold lines 44, 46, 38d, 38e, 36b.

The device 10 can be converted from a mouthpiece configuration (as shown in Figure 6) to a mask configuration (as shown in Figure 7) by cutting along one of the cut lines 20 to remove an end section of the housing 10 at the outlet opening 19 end of the device. Once separated, the side walls 14 can be squeezed inwards to cause the upper and lower walls 11, 12 to move outwardly away from each other, which is assisted by folding at fold lines fold lines 49a, 49b, 49c to shape the second end 16 such that it produces the mask configuration opening that has a significantly greater area than that of the outlet opening 19 and can be placed over to envelop a mouth and a nose of a user, as shown in Figure 7. In such a configuration, the second end forms a sealing relationship with the face of the user so that the mixture of air and medication particles can be inhaled.

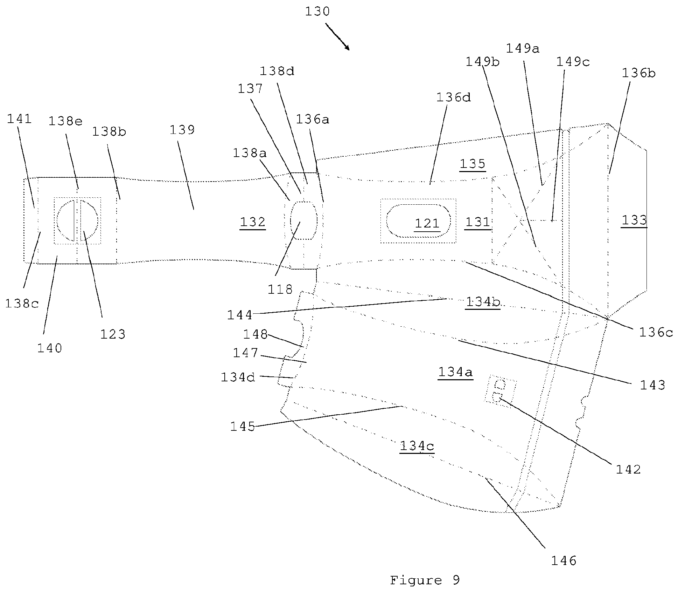

Figure 9 shows a blank 130 for making a medication inhalation device according to another, although not the only other, embodiment of the present invention.

The features of the blank 130 follow the same reference numbers as the blank 30 increased by 100.

The blank 130 functions in essentially the same manner as described for the blank 30.

The blank 130 differs from the blank 30 in that there is no end section (i.e. the section aft of the cut lines 20) of the second adjacent panel 33. As such, there is no opening that corresponds to opening 19 in the blank 30, nor are there any cut lines 20. As a consequence, in the assembled device, the second end 16 can form a mask configuration only. In other words, unlike the device assembled from the blank 30, there is no mouthpiece configuration.

It will be understood to persons skilled in the art of the invention that many modifications may be made without departing from the scope of the invention which is defined by the appended claims.

In the claims which follow, and in the preceding description, except where the context requires otherwise due to express language or necessary implication, the word "comprise" and variations such as "comprises" or "comprising" are used in an inclusive sense, i.e. to specify the presence of the stated features but not to preclude the presence or addition of further features in various embodiments of the apparatus and method as disclosed herein.

## Claims

1. A medication inhalation device comprising a housing (10) that is movable between a collapsed state and an expanded state, wherein when the housing is in the expanded state the housing defines a volume in which medication delivered from a medication delivery device can mix with air, and wherein when the housing is in the expanded state the housing comprises:
external walls (11, 12, 13, 14) that define the volume,
a first end (15) with an inlet opening (18) adapted to receive a medication from a medication delivery device for delivering the medication to the volume, and
a second end (16) with an outlet opening (19) adapted to communicate with a mouth of a user such that a mixture of air and medication can be inhaled from the volume by the user,
the second end being convertible between a mouthpiece configuration and a mask configuration,
wherein, in the mouthpiece configuration, the mouth of the user can be placed in relation to the outlet opening so that the mixture of air and medication can be inhaled, and
wherein, in the mask configuration, the outlet opening envelops the mouth and a nose of the user such that the second end forms a sealing relationship with the face of the user so that the mixture of air and medication can be inhaled.

2. The medication inhalation device of claim 1, wherein in the collapsed state the housing has a substantially flat configuration.

3. The medication inhalation device of claim 1 or claim 2, wherein the housing comprises an internal wall (22) which extends between the external walls to partition the volume into two chambers when in the expanded state, with the internal wall having an opening to allow fluid flow between the chambers.

4. The medication inhalation device of claim 3, wherein the housing has at least one one-way valve or has a one-way inhalation valve and a one-way exhalation valve.

5. The medication inhalation device of claim 4, wherein the one-way inhalation valve is located on the internal wall and the one-way exhalation valve is located on the external wall.

6. The medication inhalation device of claim 4 or claim 5, wherein the one-way inhalation valve comprises a moveable flap or wherein the one-way inhalation valve is a flutter valve.

7. The medication inhalation device of any one of the preceding claims, wherein the housing has a viewing window (21).

8. The medication inhalation device of any one of the preceding claims, wherein the device is formed from a single unitary sheet of stock.

9. The medication inhalation device of any one of the preceding claims, wherein the housing is tapered between the first end and the second end.

10. A blank for making a medication inhalation device, the blank being made from a foldable board and comprising:
a plurality of panels separated by fold lines (36a-36d, 38a-38e, 49a-49c) that allow the panels to be folded about the fold lines to form the device, with the formed device defining a housing that is movable between a collapsed state and an expanded state, and the expanded state being convertible between a mouthpiece configuration and a mask configuration, the panels comprising:
a central panel (31) and a plurality of adjacent panels to the central panel,
the adjacent panels comprising:
a first adjacent panel (32) having a first opening,
a second adjacent panel (33) having a second opening and a separable portion;
wherein the adjacent panels are foldable relative to the central panel about the fold lines and can be secured to the central panel to define external walls of a housing in the formed device,
wherein the housing defines a volume when the housing is in the expanded state of the formed device and medication can be delivered to the volume from a medication device communicating with the first opening and can mix with air and be inhaled by a user through the second opening, with the second opening forming the mouthpiece configuration of the formed device, and
wherein the separable portion can be separated from the formed device and increase the size of the second opening and form the mask configuration when the housing is in the expanded state of the formed device, with the mask configuration being able to envelop a mouth and a nose of the user and form a sealing relationship with the face of the user so that air and medication can be inhaled from the volume.

11. The blank of claim 10, wherein the second adjacent panel comprises a tongue portion having a third opening, the tongue portion being inwardly foldable about fold lines to define an internal wall which extends between, and is securable to, the external walls to partition the volume into two chambers in which the third opening allows fluid flow between the chambers.

12. The blank of claim 11, wherein the third opening has a one-way valve.

13. The blank of any one of claims 10-12, wherein the central panel has a viewing window.

14. The blank of claim 10, wherein the separable portion comprises perforations that form tear lines.

15. A method of making the medication inhalation device defined in any one of claims 1 to 9 using the blank defined in claim 10, the method comprising the steps of:
- folding the plurality of adjacent panels relative to the central panel about the fold lines;
- securing the adjacent panels to the central panel to define external walls of the housing; and
- breaking the separable portion to convert the second end from the mouthpiece configuration to the mask configuration.

## Patentansprüche

1. Medikamenteninhalationsvorrichtung, die ein Gehäuse (10) aufweist, das zwischen einem kollabiertem Zustand und einem ausgedehnten Zustand bewegbar ist, wobei, wenn das Gehäuse in dem ausgedehnten Zustand ist, das Gehäuse ein Volumen definiert, in dem sich ein von einer Medikamentenzuführvorrichtung zugeführtes Medikament mit Luft mischen kann, und wobei, wenn das Gehäuse in dem ausgedehnten Zustand ist, das Gehäuse aufweist:
Außenwände (11, 12, 13, 14), die das Volumen definieren,
ein erstes Ende (15) mit einer Einlassöffnung (18), die dazu angepasst ist, ein Medikament von einer Medikamentenzuführvorrichtung zu empfangen, zum Zuführen des Medikaments zu dem Volumen, und
ein zweites Ende (16) mit einer Auslassöffnung (19), die dazu angepasst ist, mit einem Mund eines Nutzers in Verbindung zu sein, sodass eine Mischung von Luft und Medikament von dem Volumen durch den Nutzer inhaliert werden kann,
wobei das zweite Ende zwischen einer Mundstückgestaltung und einer Maskengestaltung umwandelbar ist,
wobei in der Mundstückgestaltung der Mund des Nutzers in Beziehung zu der Auslassöffnung platziert werden kann, sodass die Mischung von Luft und Medikament inhaliert werden kann, und
wobei in der Maskengestaltung die Auslassöffnung den Mund und eine Nase des Nutzers umschließt, sodass das zweite Ende eine Dichtungsbeziehung mit dem Gesicht des Nutzers ausbildet, sodass die Mischung von Luft und Medikament inhaliert werden kann.

2. Medikamenteninhalationsvorrichtung nach Anspruch 1, wobei in dem kollabierten Zustand das Gehäuse eine im Wesentlichen flache Gestaltung hat.

3. Medikamenteninhalationsvorrichtung nach Anspruch 1 oder Anspruch 2, wobei das Gehäuse eine Innenwand (22) aufweist, die sich zwischen den Außenwänden zum Unterteilen des Volumens in zwei Kammern erstreckt, wenn es in dem ausgedehnten Zustand ist, wobei die Innenwand eine Öffnung zum Ermöglichen einer Fluidströmung zwischen den Kammern hat.

4. Medikamenteninhalationsvorrichtung nach Anspruch 3, wobei das Gehäuse mindestens ein Rückschlagventil hat oder ein Rückschlaginhalationsventil und ein Rückschlagexhalationssventil hat.

5. Medikamenteninhalationsvorrichtung nach Anspruch 4, wobei das Rückschlaginhalationsventil an der Innenwand liegt und das Rückschlagexhalationssventil an der Außenwand liegt.

6. Medikamenteninhalationsvorrichtung nach Anspruch 4 oder Anspruch 5, wobei das Rückschlaginhalationsventil eine bewegliche Klappe aufweist oder wobei das Rückschlaginhalationsventil ein Heimlich-Ventil ist.

7. Medikamenteninhalationsvorrichtung nach einem der vorangehenden Ansprüche, wobei das Gehäuse ein Sichtfenster (21) hat.

8. Medikamenteninhalationsvorrichtung nach einem der vorangehenden Ansprüche, wobei die Vorrichtung aus einem einzelnen einheitlichen Vorratsbogen ausgebildet ist.

9. Medikamenteninhalationsvorrichtung nach einem der vorangehenden Ansprüche, wobei das Gehäuse zwischen dem ersten Ende und dem zweiten Ende kegelig ist.

10. Rohling zum Anfertigen einer Medikamenteninhalationsvorrichtung, wobei der Rohling aus einem faltbaren Karton angefertigt ist und aufweist:
eine Vielzahl Paneele, die durch Faltlinien (36a - 36d, 38a - 38e, 49a - 49c) getrennt sind, die es den Paneelen ermöglichen, um die Faltlinien gefaltet zu werden, zum Ausbilden der Vorrichtung, wobei die ausgebildete Vorrichtung ein Gehäuse definiert, das zwischen einem kollabierten Zustand und einem ausgedehnten Zustand bewegbar ist, und der ausgedehnte Zustand zwischen einer Mundstückgestaltung und einer Maskengestaltung umwandelbar ist, wobei die Paneele aufweisen:
ein Zentralpaneel (31) und eine Vielzahl zu dem Zentralpaneel benachbarter Paneele,
wobei die benachbarten Paneele aufweisen:
ein erstes benachbartes Paneel (32), das eine erste Öffnung hat,
ein zweites benachbartes Paneel (33), das eine zweite Öffnung und einen abtrennbaren Abschnitt hat;
wobei die benachbarten Paneele relativ zu dem Zentralpaneel um die Faltlinien faltbar sind und an dem Zentralpaneel zum Definieren von Außenwänden eines Gehäuses in der ausgebildeten Vorrichtung fixiert werden können,
wobei das Gehäuse ein Volumen definiert, wenn das Gehäuse in dem ausgedehnten Zustand der ausgebildeten Vorrichtung ist, und ein Medikament zu dem Volumen von einer Medikamentenvorrichtung zugeführt werden kann, die mit der ersten Öffnung in Verbindung ist, und sich mit Luft mischen kann und durch einen Nutzer durch die zweite Öffnung inhaliert werden kann, wobei die zweite Öffnung die Mundstückgestaltung der ausgebildeten Vorrichtung ausgebildet, und
wobei der abtrennbare Abschnitt von der ausgebildeten Vorrichtung abgetrennt werden kann und die Größe der zweiten Öffnung erhöhen kann und die Maskengestaltung ausbilden kann, wenn das Gehäuse in dem ausgedehnten Zustand der ausgebildeten Vorrichtung ist, wobei die Maskengestaltung dazu fähig ist, einen Mund und eine Nase des Nutzers zu umschließen und eine Dichtungsbeziehung mit dem Gesicht des Nutzers auszubilden, sodass Luft und Medikament von dem Volumen inhaliert werden können.

11. Rohling nach Anspruch 10, wobei das zweite benachbarte Paneel einen Zungenabschnitt aufweist, der eine dritte Öffnung hat, wobei der Zungenabschnitt um Faltlinien einwärts faltbar ist, zum Definieren einer Innenwand, die sich zwischen den Außenwänden erstreckt und an diesen fixierbar ist, zum Unterteilen des Volumens in zwei Kammern, bei denen die dritte Öffnung eine Fluidströmung zwischen den Kammern ermöglicht.

12. Rohling nach Anspruch 11, wobei die dritte Öffnung ein Rückschlagventil hat.

13. Rohling nach einem der Ansprüche 10 bis 12, wobei das Zentralpaneel ein Sichtfenster hat.

14. Rohling nach Anspruch 10, wobei der abtrennbare Abschnitt Perforationen aufweist, die Reißlinien ausbilden.

15. Verfahren zum Anfertigen der Medikamenteninhalationsvorrichtung, die in einem der Ansprüche 1 bis 9 definiert ist, unter Verwendung des Rohlings, der in Anspruch 10 definiert ist, wobei das Verfahren die folgenden Schritte aufweist:
- Falten der Vielzahl benachbarter Paneele relativ zu dem Zentralpaneel um die Faltlinien;
- Fixieren der benachbarten Paneele an dem Zentralpaneel zum Definieren von Außenwänden des Gehäuses; und
- Brechen des abtrennbaren Abschnitts zum Umwandeln des zweiten Endes von der Mundstückgestaltung zu der Maskengestaltung.

## Revendications

1. Dispositif d'inhalation de médicament comprenant un boîtier (10) qui est mobile entre un état replié et un état déployé, où lorsque le boîtier est à l'état déployé, le boîtier définit un volume dans lequel un médicament administré depuis un dispositif d'administration de médicament peut se mélanger avec de l'air, et où lorsque le boîtier est à l'état déployé le boîtier comprend :
des parois externes (11, 12, 13, 14) qui définissent le volume,
une première extrémité (15) comportant une ouverture d'entrée (18) adaptée pour recevoir un médicament provenant d'un dispositif d'administration de médicament pour administrer le médicament dans le volume, et
une seconde extrémité (16) comportant une ouverture de sortie (19) adaptée pour communiquer avec une bouche d'un utilisateur de sorte qu'un mélange d'air et de médicament puisse être inhalé du volume par l'utilisateur,
la seconde extrémité étant convertible entre une configuration d'embout buccal et une configuration de masque,
où, dans la configuration d'embout buccal, la bouche de l'utilisateur peut être placée par rapport à l'ouverture de sortie pour que le mélange d'air et de médicament puisse être inhalé, et
où, dans la configuration de masque, l'ouverture de sortie enveloppe la bouche et un nez de l'utilisateur de sorte que la seconde extrémité forme une relation d'étanchéité avec le visage de l'utilisateur afin que le mélange d'air et de médicament puisse être inhalé.

2. Dispositif d'inhalation de médicament selon la revendication 1, où à l'état replié le boîtier a une configuration sensiblement plate.

3. Dispositif d'inhalation de médicament selon la revendication 1 ou la revendication 2, où le boîtier comprend une paroi interne (22) qui s'étend entre les parois externes pour diviser le volume en deux chambres lorsqu'il est à l'état déployé, la paroi interne ayant une ouverture pour permettre le flux de fluide entre les chambres.

4. Dispositif d'inhalation de médicament selon la revendication 3, où le boîtier a au moins une soupape unidirectionnelle ou une soupape d'inhalation unidirectionnelle et une soupape d'expiration unidirectionnelle.

5. Dispositif d'inhalation de médicament selon la revendication 4, où la soupape d'inhalation unidirectionnelle est située sur la paroi interne et la soupape d'expiration unidirectionnelle est située sur la paroi externe.

6. Dispositif d'inhalation de médicament selon la revendication 4 ou la revendication 5, où la soupape d'inhalation unidirectionnelle comprend un clapet mobile ou, où la soupape d'inhalation unidirectionnelle est une soupape oscillante (« *flutter valve* »).

7. Dispositif d'inhalation de médicament selon l'une quelconque des revendications précédentes, où le boîtier a une fenêtre d'observation (21).

8. Dispositif d'inhalation de médicament selon l'une quelconque des revendications précédentes, où le dispositif est formé à partir d'une seule feuille unitaire de matière.

9. Dispositif d'inhalation de médicament selon l'une quelconque des revendications précédentes, où le boîtier est conique entre la première extrémité et la seconde extrémité.

10. Ébauche pour la fabrication d'un dispositif d'inhalation de médicament, l'ébauche étant réalisée à partir d'une plaque pliable et comprenant :
une pluralité de panneaux séparés par les lignes de pliage (36a-36d, 38a-38e, 49a-49c) qui permettent de plier les panneaux autour des lignes de pliage pour former le dispositif, avec le dispositif formé définissant un boîtier qui est mobile entre un état replié et un état déployé, et l'état déployé étant convertible entre une configuration d'embout buccal et une configuration de masque, les panneaux comprenant :
un panneau central (31)
et une pluralité de panneaux adjacents au panneau central,
les panneaux adjacents comprenant:
un premier panneau adjacent (32) ayant une première ouverture,
un second panneau adjacent (33) ayant une seconde ouverture et une partie séparable ;
où les panneaux adjacents sont pliables par rapport au panneau central autour des lignes de pliage et peuvent être fixés au panneau central pour définir des parois externes d'un boîtier dans le dispositif formé,
où le boîtier définit un volume lorsque le boîtier est dans l'état déployé du dispositif formé et un médicament peut être administré dans le volume depuis un dispositif d'administration de médicament communiquant avec la première ouverture et peut se mélanger avec de l'air et être inhalé par un utilisateur à travers la seconde ouverture, avec la seconde ouverture formant la configuration d'embout buccal du dispositif formé, et
où la partie séparable peut être séparée du dispositif formé et augmenter la taille de la seconde ouverture et former la configuration de masque lorsque le boîtier est dans l'état déployé du dispositif formé, la configuration de masque étant apte à envelopper une bouche et un nez de l'utilisateur et à former une relation d'étanchéité avec le visage de l'utilisateur afin que de l'air et un médicament puissent être inhalés du volume.

11. Ébauche selon la revendication 10, où le second panneau adjacent comprend une partie de languette ayant une troisième ouverture, la partie de languette étant pliable vers l'intérieur autour de lignes de pliage pour définir une paroi interne qui s'étend entre les parois externes et est fixée aux parois externes pour diviser le volume en deux chambres dans lesquelles la troisième ouverture permet le flux de fluide entre les chambres.

12. Ébauche selon la revendication 11, où la troisième ouverture a une soupape unidirectionnelle.

13. Ébauche selon l'une quelconque des revendications 10-12, où le panneau central a une fenêtre d'observation.

14. Ébauche selon la revendication 10, où la partie séparable comprend des perforations qui forment des lignes de déchirure.

15. Procédé pour la fabrication du dispositif d'inhalation de médicament défini dans l'une quelconque des revendications 1 à 9 à l'aide de l'ébauche définie dans la revendication 10, le procédé comprenant les étapes suivantes :
- plier la pluralité de panneaux adjacents par rapport au panneau central autour des lignes de pliage ;
- fixer les panneaux adjacents au panneau central pour définir des parois externes du boîtier ; et
- rompre la partie séparable pour convertir la seconde extrémité de la configuration d'embout buccal à la configuration de masque.
